# EUROPEAN PATENT SPECIFICATION

(11) **EP 4 178 481 B1**
(45) Date of publication and mention of the grant of the patent: **09.10.2024**
(21) Application number: 21748793.3
(22) Date of filing: 07.07.2021
(51) Int. Cl.: A61B 46/20, A61B 46/23

(54) **PROTECTIVE BARRIER FOR FACE ISOLATION IN OROTRACHEAL MANEUVERS**
SCHUTZBARRIERE ZUR GESICHTSISOLIERUNG BEI OROTRACHALEN MANÖVERN
BARRIÈRE DE PROTECTION POUR ISOLATION FACIALE DANS DES MANOEUVRES OROTRACHÉALES

(30) Priority: 07.07.2020 AR P200101921
(43) Date of publication of application: 17.05.2023
(73) Proprietor: Spitale, Luis Santos, Córdoba, 5014 (AR); Siebenhaar, Guillermo Leonardo, Provincia de Cordoba, 5014 (AR)
(72) Inventor: SIEBENHAAR, Guillermo Leonardo, Ciudad de Córdoba Provincia de Córdoba, 5014 (AR)
(74) Representative: Isern Patentes y Marcas S.L.
(86) International application number: PCT/EP2021/068762
(87) International publication number: WO 2022/008564

(56) References cited:
- CN-U- 203 122 636
- US-A- 6 055 987
- US-A1- 2008 236 598

## Description

### Field of the invention

The present invention pertains to the field of sanitary protection of patients and healthcare practitioners. It particularly refers to protections capable of generating isolating barriers between patients and practitioners, more particularly to the barriers that should be established under circumstances where there is a high possibility of infection and there are no negative pressure facilities or other means of control.

### Background

Various means are known and have been proposed for avoiding or at least minimizing the risks of infection of healthcare staff in contact with potentially contagious patients.

In order to avoid this, various alternatives have been proposed for isolating practitioners from patients that may be contagious during maneuvers, especially those related to orotracheal interventions.

During these practices there is an important contact between patients and healthcare professionals so that the aerosols normally produced during orotracheal intubations or extubations constitute an inevitable vehicle of infection.

In fact, if the patient coughs during an airway intervention, droplets of various sizes are generated. The smallest droplets, i.e. < 5 µm, turn into aerosols that become suspended and then travel through the air. Further, the water content of the small droplets evaporates, thus reducing their size making them even smaller. This allows them to travel over greater distances as they are capable of remaining in suspension for longer periods.

The most complex means of protection are negative pressure facilities where the aerosols are continuously forced out by suction means, such as fans or suckers, towards an exit to the outside and in addition they may be filtered to avoid contaminating the environment.

The most simple means are boxes or blankets which are used for separating the patients from the attending physicians and other practitioners.

These isolating means are placed between patients and physicians, and are temporary barriers for situations where appropriate protecting means are absent.

Generally, these means of protection are not properly adjusted and must be customized for a particular procedure on the spur of the moment, thus generating risks of infection.

US 2008/236598 A1 discloses a drape for covering a patient during a medical procedure.

Therefore portable barrier means would be desirable for any situation related to orotracheal intubations or extubations which should be previoulsy adjusted as suitable barriers providing easy fit and good visibility, which would allow the professional to maneuver adequately during this type of procedure, in addition to being portable and being an effective barrier to control the contaminating aerosols produced during maneuvers carried out by intervening health professionals and auxiliaries.

### Summary of the Invention

Accordingly, the object of the present invention is to provide a protective barrier for face isolation in orotracheal maneuvers, comprising:
an essentially rectangular sheet made of a clear plastic material comprising, on its back side, a plurality of first circumferentially arranged fastening means and second cross-sectionally arranged fastening means substantially dividing the sheet in half thereby defining two sections; and
at least four sleeves made of a clear plastic material affixed to the sheet in one of the sections defined by the second cross-sectionally arranged fastening means defining an outwardly opening arc, wherein said sleeves comprise third fastening means arranged internally and circumferentially on the distal ends of each of the sleeves.

Preferably, the plastic material is selected from polypropylene, polyethylene, crystal polyvinyl chloride, and copolymers thereof.

More preferably, the sheet and the sleeves have a thickness from 50 to 100 microns.

In a preferred embodiment, the first fastening means circumferentially arranged on the sheet comprise at least two strips made of an adhesive material and the second cross-sectionally arranged fastening means comprise at least one strip made of an adhesive material.

Likewise, the third fastening means arranged internally and circumferentially on the distal ends of each of the sleeves comprise at least one strip of an adhesive material.

In a further preferred embodiment, the adhesive material is an hypoallergenic contact adhesive protected by a detachable protecting tape.

In a still more preferred embodiment, the detachable protecting tape is made of a plastic material.

Particularly, the hypoallergenic adhesive is an hypoallergenic adhesive made of pressure sensitive acrylate.

Preferably, the sleeves comprise a longitudinally-welded flat film.

More preferably, the longitudinal welding of the sleeves comprises heat welding.

Even more preferably, the sleeves are attached to the sheet by heat welding.

In a preferred embodiment, the sheet is 100 to 140 cm long and 70 to 90 cm wide; the adhesive strips are 1 to 5 cm wide and are spaced 15 to 25 cm apart from each other; central sleeves are placed at a distance of 25 to 35 cm from the nearest border that defines the width of the sheet, measured from the center of the sleeve; lateral sleeves are placed at a distance of 20 to 30 cm from the nearest border that defines the width of the sheet, measured from the center of each sleeve; the distance between centers of the central sleeves is of 5 to 15 cm; the distance between the centers of the lateral sleeves is of 20 to 30 cm; and the two sections of the sheet defined by the second cross-sectionally arranged fastening means are 70 to 90 cm long and 50 to 70 cm wide; and the sleeves have a diameter of 4 to 6 cm and a length of 25 to 35 cm.

In a preferred embodiment, the sheet is 120 cm long ; 80 cm wide; the adhesive strips are 3 cm wide and are spaced 20 cm apart from each other; central sleeves are placed at a distance of 30 cm to the nearest border that defines the width of the sheet, measured from the center of the sleeve; lateral sleeves are placed at a distance of 25 cm from the center of each sleeve to the nearest border that defines the width of the sheet; the distance between the centers of the central sleeves is 8 cm; the distance between the centers of the lateral sleeves is 24 cm; and the two sections of the sheet defined by the second cross-sectionally arranged fastening means are 80 cm long and a width of 60 cm; and the sleeves have a diameter of 5 cm and a length of 30 cm.

Additionally, the protective barrier described herein comprises an elastomeric stopper for blocking the tube-end opening of the endotracheal tube used in orotracheal maneuvers.

### Brief description of the drawings

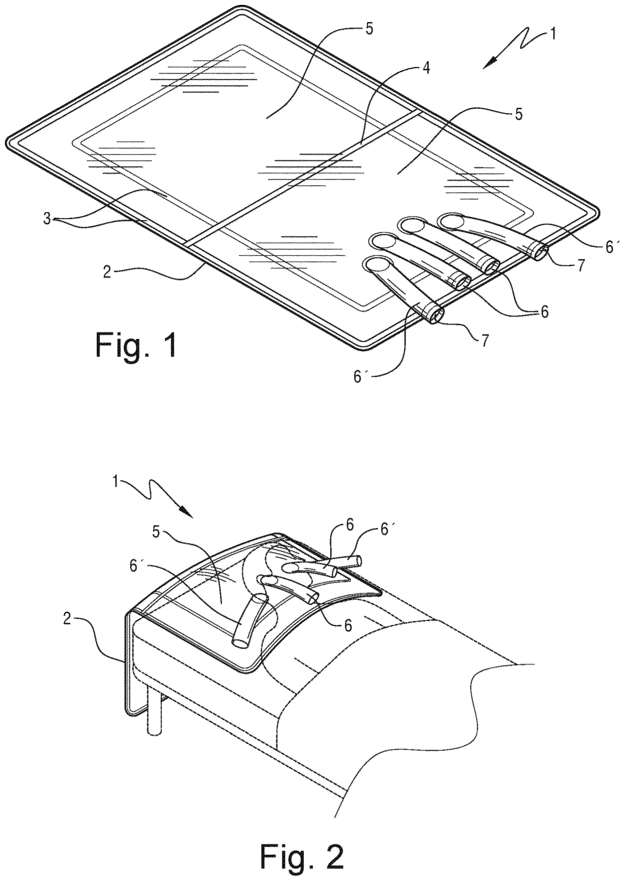

Figure 1 shows a perspective of a preferred embodiment of the protective barrier for face isolation in orotracheal maneuvers of the present invention, wherein the sheet and the sleeves are unfolded.
Figure 2 is a perspective view of the protective barrier for face isolation in orotracheal maneuvers of Figure 1, which is applied in an unfolded form on a patient in need thereof.

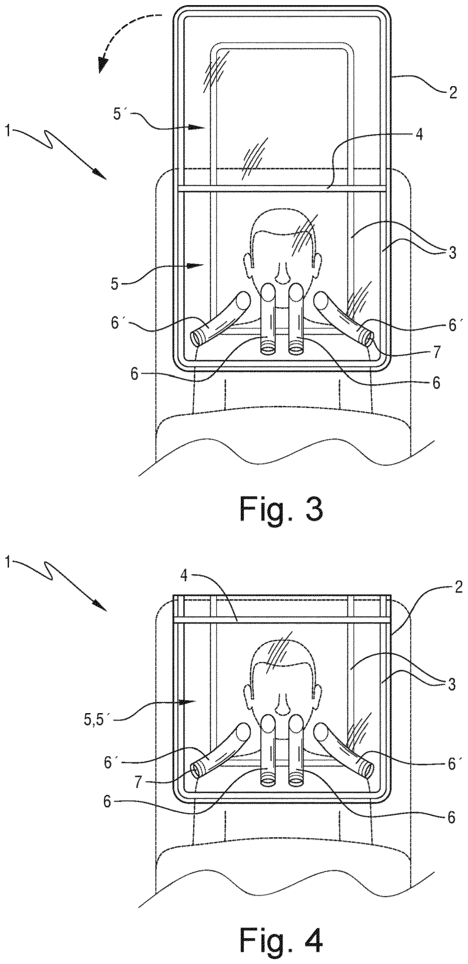

Figure 3 is a top view of the protective barrier for face isolation in orotracheal maneuvers of Figure 1, which is applied unfolded on a patient in need thereof.
Figure 4 is a top view of the protective barrier for face isolation in orotracheal maneuvers of Figure 1, which is applied folded on a patient in need thereof.
Figure 5 is a schematic view of the arrangement of a video laryngoscope and an endotracheal tube arranged in respective sleeves of the protective barrier of the present invention to carry out an orotracheal maneuver.
Figure 6 shows an elastomeric or silicone rubber elastic stopper having an internal diameter of about 15 mm capable of fitting into any opening of the endotracheal tubes

### Detailed description of the invention

In those cases where a patient suspected of being a carrier of a disease transmissible by aerosols must undergo a procedure, for example, an orotracheal intubation to connect the patient to a mechanical ventilatot and/or a respirator, it is imperative to preserve the healthcare practitioners by isolating the patient during manipulation of the airways, given that as it is during the orotracheal procedure that most aerosols and saliva microparticles present in those aerosols, as carriers of highly contagious pathogenic agents, are dispersed. Then, closed protective systems are required in order to adequately and completely isolate the patient.

The highly contagious and dangerous diseases that can spread during these orotracheal manipulations comprise, among others: influenza A (also known as H1N1 or swine), avian influenza, pneumococcal diseases (including pneumococcal pneumonia, pneumococcal meningitis and pneumococcal bacteremia), Severe Acute Respiratory Syndromes known by its acronym SARS, and further more particularly the highly contagious Covid-19 virus that has unleashed a pandemic that has largely exceeded the capacity of the health facilities of most countries all over the world, and has caused numerous deaths.

In medicine, intubation refers to a method by means of which a tube is inserted through an external or internal orifice of the body, being natural or artificial. This term is related to the word endoscopy, but is commonly used to refer to a tracheal intubation. This tracheal intubation consists in introducing a flexible plastic tube through the trachea of a patient to protect the upper airways and thereby provide the required means for mechanical ventilation. An endotracheal intubation may be performed orally or through the nose of the patient. Endotracheal intubation is used for controlling patients' airways as may be necessary depending on their condition. An endotracheal intubation may require the use of neuromuscular blockade or induction.

Further, once the patient has recovered, the artificial airway is removed to allow the patient to breathe autonomously. This maneuver is known as extubation, which consists in removing an endotracheal tube, whereas a decannulation consists in removing a tracheostomy cannula.

In this scenario, the availability of means for preserving the health of the intervening health carers becomes imperative, specially when there are no sufficient negative pressure facilities in health centers, such as hospitals, health centers or clinics, when medical assistance is performed in a mobile environment, such as an ambulance, or at the home or any place where the patient may be, or if the health centers are overwhelmed by a high exceptional influx of contagious patients needing attention and suitable traditional protection means are not available for protecting the attending health care personnel against possible infections.

Thus, the object of the present invention is to provide a protective barrier (1) for face isolation in orotracheal maneuvers, comprising:
an essentially rectangular sheet (2) made of a clear plastic material comprising, on its back side, a plurality of first circumferentially arranged fastening means (3) and second cross-sectionally arranged fastening means (4) substantially dividing the sheet (2) in half and thereby defining two sections (5, 5'); and
at least four sleeves (6, 6') made of a clear plastic material affixed to the sheet (2) in one of the sections (5) defined by the second cross-sectionally arranged fastening means (4) defining an outwardly opening arc, wherein said sleeves (6, 6') comprise third fastening means (7) arranged internally and circumferentially within the distal ends of each of the sleeves (6, 6').

The plastic material used for manufacturing the protective barrier (1) for face isolation in orotracheal maneuvers is selected from polypropylene, polyethylene, crystal polyvinyl chloride, and copolymers thereof. In preferred embodiments, these materials are of a high density that provides more transparency.

This plastic material must be sufficiently tear-resistant to prevent breakage of the sheet (2) or the sleeves (6, 6') of the protective barrier (1) during the orotracheal maneuvers performed on the patient, but at the same time they should be suitably flexible to allow the health care professionals to operate the necessary instruments and supplies during manipulation of the upper airways without impediments and as naturally as possible.

Likewise, the plastic material must be transparent for allowing seeing through it, so that the health practitioner may perform the orotracheal maneuvers without inconveniencies, avoiding at the same time additional impairments for the patient since it is important to view the structures that are being worked on.

In a preferred embodiment, the sheet (2) and the sleeves (6, 6') have a thickness of 50 to 100 microns, which confer the desired mechanical resistance to the protective barrier (1).

To provide adequate isolation, the first circumferentially arranged fastening means (3) on the sheet (2) of the protective barrier (1) of the present invention are comprising at least two strips of adhesive material and the second cross-sectionally arranged fastening means (4) are comprising at least one strip of adhesive material.

The at least two strips of adhesive material allow for adhering the perimeter of the protective barrier (1) to the stretcher or, alternatively, to fold it under the patient around the head and upper part of the thorax forming a sac by folding sheet section (5'), which has no sleeves, of sheet (2) of the protective barrier (1) under the patient, and joining together the borders of the two sections (5, 5') to each other.

Preferably, the adhesive material is an hypoallergenic contact adhesive protected by a detachable protecting tape. More preferably, the detachable protecting tape is made of a plastic material.

Likewise, in a preferred embodiment, the hypoallergenic adhesive is an hypoallergenic adhesive made of pressure sensitive acrylate.

In a preferred embodiment, sleeves (6, 6') comprise a longitudinally welded flat film to form cylindrical or slightly conical sleeves. Similarly, the longitudinal attachment of sleeves (6, 6') is made by heat weld.

More preferably, the sleeves (6, 6') are affixed to the sheet (2) by heat welding. This welding may be performed between the perimetral borders of the tube-end openings receiving sleeves (6, 6') and the proximal borders of said sleeves (6, 6'). Alternatively, a plurality of longitudinal cuts may be practiced on the proximal border of sleeves (6, 6') such that wedges are formed that may be folded on the back side of sheet (2) of the protective barrier (1) for face isolation and welded in that position to fix sleeves (6, 6') to said sheet (2).

The distal borders of sleeves (6, 6') also have internal third fastening means (7) comprising at least one strip of adhesive material protected by a detachable protecting tape that allows for introducing instruments and supplies, such as a laryngoscope, an endotracheal tube, an airway suction tube, a nasogastric tube and even a tracheostomy cannula, such that said sleeves (6, 6') allows for telescoping instruments and supplies through, introducing and pulling the required instruments according to the methodology to be used in view of the particular needs of each patient, with different rotation angles for proper manipulation and achieving at the same time the isolation of instruments by closing the respective sleeve (6, 6') on the surface of said instruments and supplies by means of the above mentioned adhesive placed internally on the borders of said sleeves (6, 6'), thus keeping instruments and supplies isolated and sterile.

The protective barrier (1) of the invention may be used in an intubation, but its use is also intended for patients who have to remain isolated for a couple of days until a favorable evolution is observed, approximately for 24, 48 or more hours, and once stabilized it can be used for extubating the patient, which is another instance of manipulation of the patient's upper airways where aerosols are generated.

In a preferred embodiment of the invention, the sheet (2) of the protective barrier (1) is from 100 to 140 cm long and from 70 to 90 cm wide; the adhesive strips are from 1 to 5 cm wide and are spaced 15 to 25 cm apart from each other; the central sleeves (6) are placed at a distance of 25 to 35 cm from the nearest border that defines the width of the sheet; measured from the center of the sleeve; the lateral sleeves (6') are placed at a distance of 20 to 30 cm from the nearest border that defines the width of the sheet the center of each sleeve; the distance between the centers of the central sleeves (6) is of 5 to 15 cm; the distance between the centers of the lateral sleeves (6') is of 20 to 30 cm; and the two sections (5, 5') of the sheet defined by the second cross-sectionally arranged fastening means (4) are 70 to 90 cm long and 50 to 70 cm wide; and the sleeves (6, 6') have a diameter of 4 to 6 cm and a length of 25 a 35 cm.

In a further preferred embodiment, the sheet (2) of the protective barrier (1) is 120 cm long and 80 cm wide; the adhesive strips are 3 cm wide and are spaced 20 cm apart from each other; the central sleeves (6) are placed at a distance of 30 cm from the center of the sleeve to the nearest border that defines the width of the sheet; the lateral sleeves (6') are placed at a distance of 25 cm from the center of each sleeve to the nearest border that defines the width of the sheet; the distance between the centers of central sleeves (6) is of 8 cm; the distance between the centers of the lateral sleeves (6') is of 24 cm; the two sections (5, 5') of the sheet defined by the second cross-sectionally arranged fastening means (4) are 80 cm long and 60 cm wide; and the sleeves (6, 6') have a diameter of 5 cm and a length of 30 cm.

Endotracheal intubation is a maneuver of common practice carried out by anesthesiologists and physicians. Under circumstances in which the patient may be a carrier of a transmissible microorganism through aerosols formed in the upper respiratory tract, particularly the highly contagious and lethal Covid-19 virus, the use of a protective barrier for face isolation (1) like that of the present invention is essential, which makes it a supply or input of great value and importance for the protection of the health personnel.

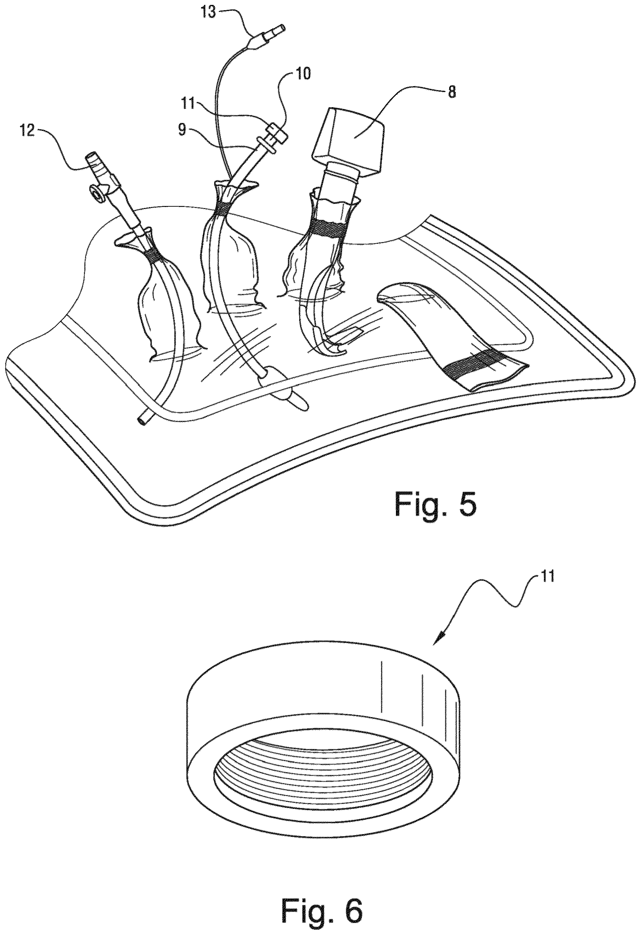

Figure 5 is a schematic representation of an arrangement of a video laryngoscope (8) and an endotracheal tube (9), both shown within respective sleeves (6, 6') of the protective barrier (1) of the present invention, wherein the connecting orifice of said endotracheal tube (9) is plugged by a stopper (11) made of an elastic material to close the opening through which contaminating aerosols could escape during the intubation maneuver.

The intubation system comprises a video laryngoscope (8), a saliva suction tube (12), a protective barrier (1) as described herein, an endotracheal tube (9), and an elastic stopper (11) for the tube-end opening (10) connecting the endotracheal tube (9) with an air generator. Additionally, the remaining sleeve may be used for a tube connecting to a preoxygenation mask, a nasogastric tube or any other required input as needed.

Figure 5 shows these elements together with a plug (13) of the endotracheal tube (9) that is connected to an air generator for injecting air into the balloon placed at the end of the endotracheal tube (9) and thus obturating the trachea and isolating it from permeations.

Figure 6 shows that said elastomeric or silicone rubber elastic stopper (11) has an inner bore in the order of 15 mm and fits in all the connection orifices (10) of the endotracheal tubes (9) to avoid leakage of aerosols.

## Claims

1. A protective barrier (1) for face isolation in orotracheal maneuvers **characterized by** comprising:
an essentially rectangular sheet (2) made of a clear plastic material comprising, on its back side, a plurality of first circumferentially arranged fastening means (3) and second cross-sectionally arranged fastening means (4) substantially dividing the sheet (2) in half and defining two sections (5, 5'); and
at least four sleeves (6, 6') made of a clear plastic material affixed to the sheet (2) in one of the sections (5) defined by the second cross-sectionally arranged fastening means (4) defining an outwardly opening arc, wherein said sleeves (6, 6') comprise third fastening means (7) arranged internally and circumferentially on the distal ends of each of the sleeves (6, 6').

2. The protective barrier (1) of claim 1, **characterized in that** the plastic material is selected from polypropylene, polyethylene, crystal polyvinyl chloride, and copolymers thereof.

3. The protective barrier (1) of claim 1 or 2, **characterized in that** the sheet (2) and the sleeves (6, 6') have a thickness of 50 to 100 microns.

4. The protective barrier (1) of any of the preceding claims, **characterized in that** the first circumferentially arranged fastening means (3) on the sheet comprise at least two strips of an adhesive material and the second cross-sectionally arranged fastening means (4) comprise at least one strip of an adhesive material.

5. The protective barrier (1) of any of the preceding claims, **characterized in that** the third fastening means (7) arranged internally and circumferentially on the distal ends of each of the sleeves comprises at least one strip of adhesive material.

6. The protective barrier (1) of claim 5, **characterized in that** the adhesive material is an hypoallergenic contact adhesive protected by a detachable protecting tape.

7. The protective barrier (1) of claim 6, **characterized in that** the detachable protecting tape is made of a plastic material.

8. The protective barrier (1) of claim 6 or 7, **characterized in that** the hypoallergenic adhesive is an hypoallergenic adhesive made of pressure-sensitive acrylate.

9. The protective barrier (1) of any of the preceding claims, **characterized in that** the sleeves (6, 6') comprise a longitudinally welded flat film.

## Patentansprüche

1. Schutzbarriere (1) zur Gesichtsisolierung bei orotrachealen Manövern, **dadurch gekennzeichnet, dass** sie umfasst:
eine im Wesentlichen rechteckige Folie (2) aus einem klaren Kunststoffmaterial, die auf ihrer Rückseite eine Vielzahl von ersten, in Umfangsrichtung angeordneten Befestigungsmitteln (3) und zweiten, im Querschnitt angeordneten Befestigungsmitteln (4) aufweist, die die Folie (2) im Wesentlichen in zwei Hälften teilen und zwei Abschnitte (5, 5') definieren; und
mindestens vier Hülsen (6, 6') aus einem durchsichtigen Kunststoffmaterial, die an der Folie (2) in einem der Abschnitte (5) befestigt sind, die durch die im Querschnitt angeordneten zweiten Befestigungsmittel (4) definiert sind, die eine nach außen gerichtete Öffnung bilden, wobei diese Hülsen (6, 6') dritte Befestigungsmittel (7) umfassen, die innen und in Umfangsrichtung an den distalen Enden jeder der Hülsen (6, 6') angeordnet sind.

2. Schutzbarriere (1) nach Anspruch 1, **dadurch gekennzeichnet, dass** das Kunststoffmaterial aus Polypropylen, Polyethylen, kristallinem Polyvinylchlorid und Copolymeren davon ausgewählt ist.

3. Schutzbarriere (1) nach Anspruch 1 oder 2, **dadurch gekennzeichnet, dass** die Folie (2) und die Hülsen (6, 6') eine Dicke von 50 bis 100 Mikrometern aufweisen.

4. Schutzbarriere (1) nach einem der vorhergehenden Ansprüche, **dadurch gekennzeichnet, dass** die ersten in Umfangsrichtung angeordneten Befestigungsmittel (3) an der Folie mindestens zwei Streifen aus einem Klebematerial und die zweiten im Querschnitt angeordneten Befestigungsmittel (4) mindestens einen Streifen aus einem Klebematerial umfassen.

5. Schutzbarriere (1) nach einem der vorhergehenden Ansprüche, **dadurch gekennzeichnet, dass** das dritte Befestigungsmittel (7), das innen und in Umfangsrichtung an den distalen Enden jeder der Hülsen angeordnet ist, mindestens einen Streifen aus einem Klebematerial umfasst.

6. Schutzbarriere (1) nach Anspruch 5, **dadurch gekennzeichnet, dass** das Klebematerial ein hypoallergener Kontaktkleber ist, der durch ein abnehmbares Schutzband geschützt ist.

7. Schutzbarriere (1) nach Anspruch 6, **dadurch gekennzeichnet, dass** das abnehmbare Schutzband aus einem Kunststoffmaterial hergestellt ist.

8. Schutzbarriere (1) nach Anspruch 6 oder 7, **dadurch gekennzeichnet, dass** der hypoallergene Klebstoff ein hypoallergener Klebstoff aus druckempfindlichem Acrylat ist.

9. Schutzbarriere (1) nach einem der vorhergehenden Ansprüche, **dadurch gekennzeichnet, dass** die Hülsen (6, 6') aus einer längsgeschweißten Kunststofffolie bestehen.

## Revendications

1. Barrière de protection (1) pour l'isolation du visage lors de manoeuvres orotrachéales, **caractérisée en ce qu'**elle comprend :
une feuille essentiellement rectangulaire (2) constituée d'un matériau plastique transparent comprenant, sur son côté arrière, une pluralité de premiers moyens de fixation (3) agencés de manière circonférentielle et de deuxièmes moyens de fixation agencés en coupe transversale (4) divisant sensiblement la feuille (2) dans la moitié, définissant ainsi deux sections (5, 5') ; et
au moins quatre manchons (6, 6') constitués d'un matériau plastique transparent fixés à la feuille (2) dans l'une des sections (5) définies par les deuxièmes moyens de fixation (4) agencés en coupe transversale définissant un arc ouvert vers l'extérieur, lesdits manchons (6, 6') comprenant des troisièmes moyens de fixation (7) agencés intérieurement et circonférentiellement sur les extrémités distales de chacun des manchons (6, 6').

2. Barrière protectrice (1) selon la revendication 1, **caractérisée en ce que** le matériau plastique est choisi parmi le polypropylène, le polyéthylène, le polychlorure de vinyle cristallin et leurs copolymères.

3. Barrière de protection (1) selon la revendication 1 ou 2, **caractérisée en ce que** la feuille (2) et les manchons (6, 6') ont une épaisseur de 50 à 100 microns.

4. Barrière de protection (1) selon l'une quelconque des revendications précédentes, **caractérisée en ce que** les premiers moyens de fixation (3) agencés circonférentiellement sur la feuille comprennent au moins deux bandes d'un matériau adhésif et les deuxièmes moyens de fixation (4) agencés en section transversale ) comprennent au moins une bande d'un matériau adhésif.

5. Barrière de protection (1) selon l'une quelconque des revendications précédentes, **caractérisée en ce que** les troisièmes moyens de fixation (7) agencés intérieurement et circonférentiellement sur les extrémités distales de chacun des manchons comprennent au moins une bande de matériau adhésif.

6. Barrière de protection (1) selon la revendication 5, **caractérisée en ce que** le matériau adhésif est une colle de contact hypoallergénique protégée par un ruban de protection détachable.

7. Barrière de protection (1) selon la revendication 6, **caractérisée en ce que** le ruban de protection détachable est réalisé en un matériau plastique.

8. Barrière protectrice (1) selon la revendication 6 ou 7, **caractérisée en ce que** l'adhésif hypoallergénique est un adhésif hypoallergénique en acrylate sensible à la pression.

9. Barrière de protection (1) selon l'une quelconque des revendications précédentes, **caractérisée en ce que** les manchons (6, 6') comprennent un film plat soudé longitudinalement.
